# EUROPEAN PATENT APPLICATION

(11) **EP 2 584 349 A1**
(43) Date of publication of application: **24.04.2013**
(21) Application number: 11185741.3
(22) Date of filing: 19.10.2011
(51) Int. Cl.: G01N 27/22, C12Q 1/68, G01N 33/543

(54) **A method of sensing a molecule, an apparatus and a semiconductor chip therefor**

(71) Applicant: NXP B.V., 5656 AG Eindhoven (NL)
(72) Inventor: Suy, Hilco, Redhill, Surrey RH1 1DL (GB); Frederix, Filip, Redhill, Surrey RH1 1DL (GB); Steenwinckel, David, Redhill, Surrey RH1 1DL (GB); Hoofman, Romano, Redhill, Surrey RH1 1DL (GB)
(74) Representative: Hardingham, Christopher Mark

(57) **Abstract**

A semiconductor chip, apparatus, and associated method are disclosed wherein the semiconductor chip, having at least one electrode and configured as a sensor such as a biosensor, is removably attachable to a tip of a dipstick. The dipstick tip, with the attached semiconductor chip, is arranged to be dipped into a well containing an analyte. The well may be part of a micro-titre plate. The chip electrically senses the presence of a target molecule, such as a bio-molecule, in the analyte. The sensing may typically be by means of a change in a capacitance associated with the electrode which occurs in the presence of the target molecule. The apparatus may include a plurality of dipsticks and associated semiconductor chips which are sensitive for different target molecules. Alternatively or in addition, a single semiconductor chip may have a plurality, many or even many thousands of electrodes, which may be sensitive to different target molecules.

## Description

### Field of the Invention

The present invention relates to apparatus and method for sensing a target molecule. It further relates to semiconductor chips having a sensing capability and being suitable for use in such apparatus.

### Background of the Invention

Sensors which are specific to one or more target molecules and can sense for the target molecule or molecules in an analyte, are well known. For larger molecules, and in particular for bio-molecules, that is to say organic molecules or macromolecules which are associated with living organisms, such as protein or nucleic acid, sensing is typically carried out by means of either optical or electronic sensing modes. In optical sensing, an optical property of the target molecule itself is used, or an optical marker is used in which a marker molecule is attached to the target molecule and the optical property of the marker is sensed. Typically the marker molecule may fluoresce, and the presence of the marker molecule - and thus of the attached target molecule - is determined by sensing the fluorescence. In electronic sensing, an electronic property of the target molecule is sensed, or, as is typically the case for biomolecules, a receptor molecule is provided which binds specifically to the target biomolecule, and the combination is sensed; since the combination of biomolecule and bioreceptor has different electronic properties to the bioreceptor alone, the presence or absence of the bio-molecule can thereby be determined.

Such sensors, and in particular biosensors, whether using optical or electronic sensing means, require that the analyte be brought into contact with the sensor. Conventionally this has been done by means of a fluidic and more particularly a microfluidic component to transport the analyte to the sensing region. In particular in the case of electronic sensing, it is generally required that electrical contacts be kept remote from the analyte material. Furthermore, with recent developments which have resulted in significant reduction in the physical dimensions of such sensors, the microfluidic arrangement becomes increasingly important, and complex and generally expensive, as well as becoming more susceptible to leakage.

### Summary of the invention

It is an object of the present invention to provide an apparatus for sensing a target molecule, and a method for sensing a target molecule, which is inexpensive and convenient.

According to a first aspect, there is provided apparatus for sensing a target molecule and comprising: a dipstick having a tip configured to be dipped into a well; and a semiconductor chip configured as a sensor, the semiconductor chip having a first major surface having at least one electrode thereon, which electrode is sensitive to the target molecule, the semiconductor chip being removably attachable to the tip. By attaching the sensor to a dipstick, the sensor may be transported to the analyte by the dipstick. Thereby, the requirement for fluidic transport normally associated with conventional sensors may be avoided. Moreover, by providing an apparatus wherein the semiconductor chip is removably attachable to the tip, the dipstick may be reusable. In the case of a reusable dipstick, it may be reused with the same semiconductor chip; alternatively or in addition it may be reused with a different semiconductor chip. Such re-use may provide for a cost saving in cases where the semiconductor chip may only be used for a single or for a limited number of sensing operations. The well may be a fluidic container for containing a fluid, in particular the analyte. Such an electrode which is sensitive to a target molecule is commonly referred to as a functionalized electrode.

In embodiments the semiconductor chip further comprises at least one electrical contact on a second major surface opposed to the first major surface and in electrical communication with the tip. Providing the semiconductor chip with one or more electrical contacts on its second major surface, which may be the backside of the chip, allows for convenient electrical communication with the dipstick and may provide for the electrical communication to be isolated from the sensing environment. In other embodiments, one or more electrical contacts may be provided on the chip on its first major surface.

In embodiments, the electrical communication is by means of a resilient element. The resilient element may be a spring which may provide for simple and efficient attachment of the semiconductor chip to the tip.

In embodiments the apparatus further comprises a sealing element for providing a seal between the second major surface and the tip. The sealing element may ensure that electrical contacts are isolated from the analyte and thereby avoid electrical shorts which might otherwise arise from immersion of the chip and at least part of the tip into the analyte, even if the dipstick is agitated with respect to the analyte. Alternatively, the apparatus may be arranged such that only the chip is immersed or partly immersed in the analyte.

In embodiments, the semiconductor chip is removably attachable to the tip by at least one of a resilient component and a magnetic field. Although not limited thereto, it is particularly convenient if the assembly of the dipstick and semiconductor chip is compatible with so-called pick-and-place apparatus. This may provide for automated or semi-automated operation. Attachment of the semiconductor chip to the tip, by resilient means such as a spring, or by a magnetic field, is thus convenient. Other means of attachment, such as, without limitation, use of Van der Waals force, a vacuum, or friction are also envisaged and within the scope of embodiments.

The apparatus may further comprise at least one further dipstick having a respective tip and configured to be dipped into a respective further well, the well and further well being comprised in a microtitre plate. By providing an apparatus with multiple semiconductor chips, typically one attached to the respective tip of each dipstick which forms part of an array of dipsticks, typically with regular spacing therebetween, the speed or throughput of the sensing process may be increased. Moreover, by suitable choice of the spacing or pitch between dipsticks in a linear array, the apparatus may be made compatible with conventional microtitre plates which are readily available at relatively low cost. The apparatus may be dimensioned so as to be physically similar to well-known micropipette arrays, for enhanced compatibility with standardised products and equipment in current biotechnology facilities.

In embodiments in which the apparatus includes a plurality of dipsticks, the apparatus may further comprise a further semiconductor chip being removably attachable to the respective tip and having a major surface having at least one electrode thereon, which electrode is sensitive to a second target molecule which is different from the target molecule. Such apparatus is thereby suitable for sensing, at the same time, for more than one different target molecules. The speed of sensing for a multiplicity of difference target molecules may thereby be enhanced.

According to another aspect, there is provided a semiconductor module comprising a semiconductor chip configured for use in an apparatus as described above. Without limitation the semiconductor module may be an assembly comprising other components in addition to the semiconductor chip, or may be the semiconductor chip itself.

In embodiments, the, each or some of the semiconductor chip or chips may further comprise at least one further electrode on the first major surface of the semiconductor chip, the at least one further electrode being sensitive to a further target molecule which is different from the target molecule. Thereby an individual semiconductor chip may be provided which is capable of sensing for more than one different type of target molecule. The speed or throughput of sensing for a multiplicity of difference target molecules may thereby be enhanced or further enhanced.

According to a yet further aspect, there is provided a method of sensing a target molecule, the method comprising: removably attaching a semiconductor chip or module to a tip of a dipstick, the semiconductor chip or module having a first major surface having at least one electrode thereon, which electrode is sensitive to the target molecule, and a second, opposed, major surface adjoining the tip; dipping the chip into a well having an analyte therein; electrically sensing for the molecule by means of the semiconductor chip; removing the chip from the well; and detaching the semiconductor chip from the tip. The chip may be dipped into the well such that the tip is also dipped into the well and comes into contact with the analyte; in other embodiments, the chip may be dipped into the well such that the tip does not come into contact with the analyte. The chip may form part of a module which is completely or partially dipped into the analyte. Thus in some embodiments the module including the chip is partially dipped into the analyte and the tip does not come into contact with the analyte, thereby avoiding contamination of the dipstick by the analyte and simplifying cleaning the dipstick prior to re-use.

In embodiments, removably attaching a semiconductor chip or module to a tip of a dipstick comprises magnetically attaching the chip to the tip. Alternatively, removably attaching the chip or module may comprise other methods of attachment such as, without limitation, forcing the components together by resilient means or through Van der Waals force or by means of a vacuum.

In embodiments, removably attaching a semiconductor chip or module to a tip of a dipstick further comprises forming a seal, by means of a sealing element, between the second major surface and the tip.

In embodiments, detaching the semiconductor chip or module from the tip comprises urging the chip away from the tip by a pin. In other embodiments, and without limitation, detaching the semiconductor chip or module may be affected by alteration or reversal of a magnetic field or by modification or cessation of a vacuum.

In embodiments, electrically sensing for the target molecule comprises measuring a change in a capacitance associated with the electrode, in the presence of the target molecule. In other embodiments electrically sensing for the target molecule comprises measuring a change in an impedance associated with the electrode, in the presence of the target molecule.

The target molecule may be a bio-molecule.

These and other aspects of the invention will be apparent from, and elucidated with reference to, the embodiments described hereinafter.

### Brief description of Drawings

Embodiments of the invention will be described, by way of example only, with reference to the drawings, in which
Figure 1 shows at 1(a) a schematic of an apparatus according to an embodiment, and at 1(b) a schematic of apparatus according to another embodiment;
figure 2 shows a schematic of a tip and a semiconductor chip including a magnetic detachment mechanism;
figure 3 shows, at figures 3(a), 3(b) and 3(c) respectively, different arrangements for providing electrical communication between the semiconductor chip and the tip;
figure 4 shows, at figures 4(a), 4(b) and 4(c) respectively, different stages in a method of sensing according to embodiments;
figure 5 shows an apparatus having a plurality of dipsticks, dipped into a microtitre plate; and
figure 6 is a flow diagram of a method according to embodiments of the invention.

It should be noted that the Figures are diagrammatic and not drawn to scale. Relative dimensions and proportions of parts of these Figures have been shown exaggerated or reduced in size, for the sake of clarity and convenience in the drawings. The same reference signs are generally used to refer to corresponding or similar feature in modified and different embodiments

### Detailed description of embodiments

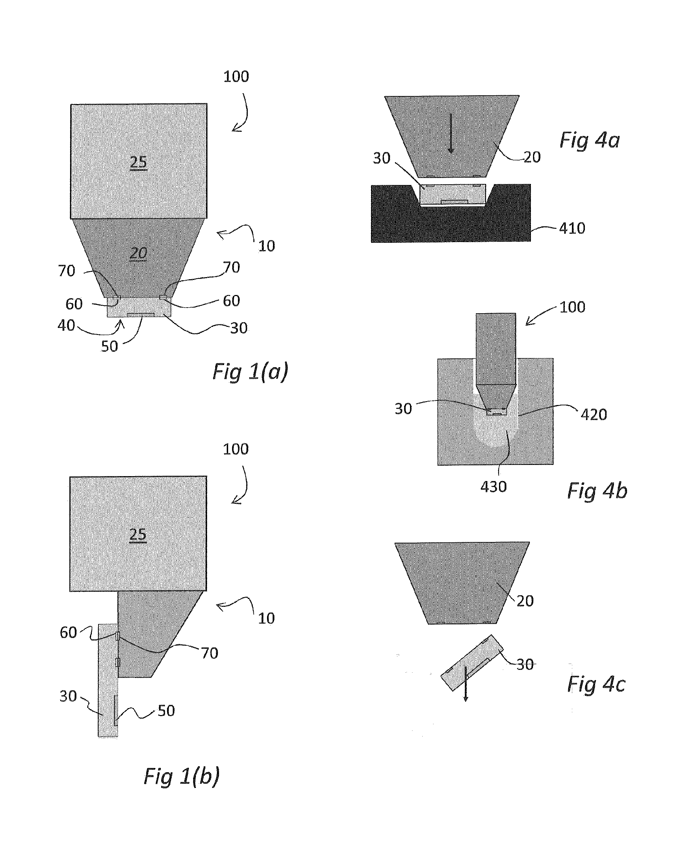

Figure 1(a) shows a schematic of an apparatus 100 according to an embodiment. The apparatus comprises a dipstick 10 having a tip 20, and a semiconductor chip 30. The dipstick 10 may be fabricated as a unitary component comprising the tip 20, or as a composite component wherein the tip 20 is separate to a stem 25. The semiconductor chip 30 has a first major surface 40. The first major surface 40 has thereon at least one electrode 50; the electrode 50 is sensitive to a target molecule. The semiconductor chip 30 includes therein electronic circuitry, not shown, which is operable to sense the presence of the target molecule bound to the electrode 50. Such semiconductor chips with one or more electrodes thereon will be familiar to the skilled person and are known from, for example, applicant's co-pending and pre-published patent application publication number W02008/1326565. It will be appreciated, that although in figure 1 a single electrode 50 is shown, in practice the semiconductor chip 13 may include a plurality of electrodes 50, and the plurality of electrodes may include up to, or even more than, an array of 128x128, or 256x256, individual electrodes. In such an array, the electrodes will typically be nano-electrodes - that is to say their lateral dimensions in the plane of the first major surface are less than or equal to 1 µm.

As shown in figure 1(a), in this embodiment the semiconductor chip has, on its second major surface which is opposed to the first major surface, one or more electrical contacts 60. The electrical contacts 60 provide electrical communication between the semiconductor chip 30 and the tip 20 of the dipstick 10. In the embodiment shown, tip 20 has corresponding electrical contacts 70 which mate with electrical contacts 60 of the semiconductor chip; however, as will be described in more detail hereinbelow, other means of providing electrical communication between the semiconductor chip and the tip may be provided, in addition to or as alternatives to either the electrical contacts 60 of the semiconductor chip 30 or the electrical contacts 70 of the tip 20, or both.

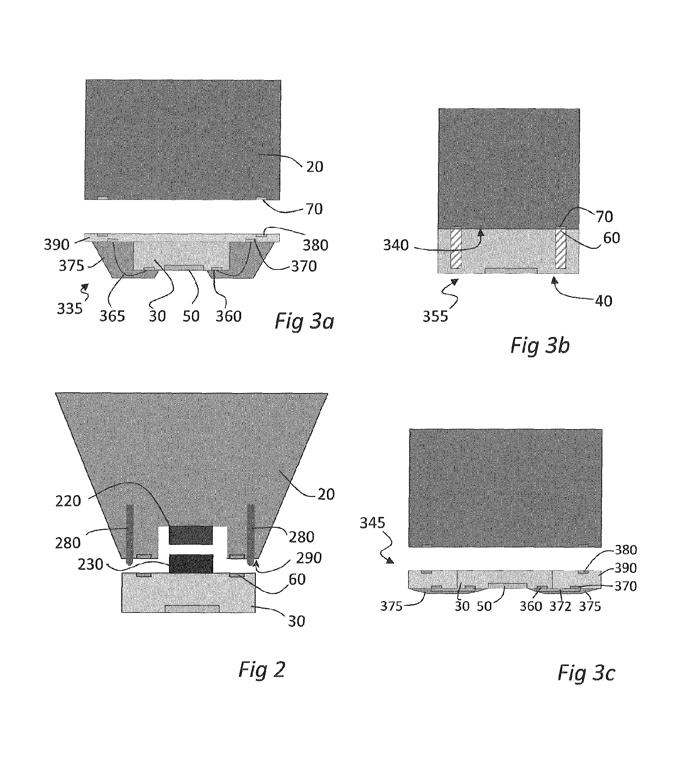

The semiconductor chip 30 may be attachable to the tip 20 by one or more of several mechanisms. Figure 2 shows an example schematic of a tip and a semiconductor chip including a magnetic attachment mechanism: in this embodiment, one or both of tip 20 and semiconductor chip 30 are provided with magnetic components 220 and 230 respectively. As shown in the figure, magnetic components 230 may be separately attached to semiconductor chip 30; in other embodiments, the magnetic components 230 may be integral within the semiconductor chip, or may be for instance a patterned metallisation on the second major surface of the chip, which may for example be formed as part of the backside metallisation process of the semiconductor chip. In the case that the chip comprises a part of a module having, in addition, a separate substrate such as a laminate, and/or encapsulant (as will be discussed in more detail hereinbelow), the magnetic components may be comprised in the module, and need not necessarily be directly in contact with the chip. Either of the magnetic components 220 and 213 may be, without limitation, permanent magnets formed from materials such as ferromagnetic materials, or may be electromagnets. Electromagnets have the advantage that attachment and detachment of the chip to the tip is particularly simple - to detach the chip from the tip, the electromagnetic field is simply switched off, and the electromagnet thus ceases to urgency components together. As a further modification or enhancement of the magnetic attachment mechanism, it will be readily appreciated by the skilled person, that one of the magnetic components 220 and 230 may be arranged as a permanent magnets, whilst the other is arranged as an electromagnet, the field of which is switchable so as to urge the chip either towards - in the case of attachment - or away - in the case of detachment - from the tip.

Other mechanisms for attachment, such as using the Van der Waals force between components 220 and 230, which in this case would be formed from suitably selected materials, may be used instead of a magnetic attachment mechanism. As a further example of such an other mechanism, a spring or clip or other resilient means may be used to secure the semiconductor chip in place. Yet another attachment mechanism may be by means of a vacuum, in which case tip 20 may be hollow or include a conduit (not shown), which is evacuated in order to provide a negative pressure between the tip and semiconductor chip to secure the semiconductor chip in place.

Although in some embodiments, the attachment may be reversible simply by removing the attaching force such as magnetic field or vacuum, in other embodiments, one or more mechanisms to effect detachment may be required. An example of such a mechanism is shown in figure 2 which includes pins 280. Tip 20 may have associated with it one or more pins 280. As shown, the pins 280 may be arranged to be extendable beyond the lower surface 290 of tip 20. Whilst the semiconductor chip 30 is attached to the tip 20, the pins 280 are retracted, so as not to project beyond the tip 20. In order to detach the semiconductor chip 30 from 20, the pins are extended to project beyond the lower surface 290 of the tip, and urge the semiconductor chip 30 away from the tip. In the case of a vacuum seal, the vacuum is thereby broken, and the chip and tip are effectively separated; in the case of a magnetic attachment, the components may be spaced far apart sufficiently far that the magnetic field is no longer sufficient to keep them together. In other embodiments, the pins 280, which may also be termed ejector pins, may be resilient, such that the chip remains attached to the tip only so long as the attaching mechanism, such as a magnetic field or vacuum as described above, is stronger than the resilience in the pins which urges the components apart.

Particularly, but without limitation, in the case that the dipstick is a unitary component, the semiconductor chip may form or comprise a semiconductor module, which may be configured to operate in a similar fashion to the removable tip of a pipette. As an example, the semiconductor chip may be integrated into a module which has a recess at its upper end, the recess having a generally inverted conical form. The upper end of the module, or its upper end in particular, may thus take the form of a sleeve. The dipstick (typically with integral tip), has a lower end have a generally conical or pyramidal protrusion. The dipstick may then be attached to the module, by means of friction, or a "push-fit" into the sleeve. Again similar to the operation of a conventional pipette, the module may be released from the dipstick by a mechanical action such as firing one or more ejector pins as discussed above.

Although as shown in figure 1(a), the chip may be arranged to be horizontal relative to a vertical dipstick, in other embodiments, the chip may be arranged to be vertical. That is, the plane of the major surface may be parallel to the elongate direction of the dipstick, rather than parallel to it as shown in figure 1(a). Such an arrangement is shown schematically in figure 1(b). The contacts 60 on the chip which provide electrical communication with electrical contacts 70 on the tip may then be on the second major surface of the chip, as in the embodiment shown in figure 1(a), or may be on the first major surface, as shown at figure 1(b). This latter arrangement is convenient, since in general the manufacturing processes required to provide the electrical contacts 60 on the first major surface may be more straightforward than those, such as the opening of so-called "through-vias", required to provide the electrical contacts 60 on the second major surface. However, in any particular application, this convenience may be offset against the additional chip area which will generally be required, in the case that the contacts are on the same major surface of the chip as the electrodes. Additional chip area may be required not only for the contacts themselves, but also to provide adequate separation between the electrodes and contacts: this additional chip area, which may also be referred to as additional semiconductor "real estate", may be particularly large in embodiments in which the contacts must be kept separate from the analyte into which the chip may be dipped, or in which no other measures (such as a fluidic seal) are used to protect the contacts from the analyte. In case the contacts must be kept separate from the analyte and no other protection is used, it may be that the chip is only partially dipped into the analyte. The separation may then required to be sufficiently large that the analyte does not "wick" up the chip to reach the contacts, which could otherwise occur particularly if the analyte has a positive contact angle with the material on the exposed surface of the chip,

Figure 3 shows, at figures 3(a), 3(b) and 3(c) respectively, different arrangements for providing electrical communication between the semiconductor chip and the tip. In figure 3(a), the semiconductor chip 30 is shown having electrical contacts 360 on its front side. The electrical contacts 360 are wire bonded to contact pads 370 by means of bond wires 365. The contact pads 370 are formed as part of a laminate component 390, onto which the semiconductor chip is bonded. Typically the chip may be partially encapsulated by encapsulant 375 which also fully encapsulates the wire bonds 365. The contact pads 370 are in electrical communication with mating contact pads 380 on the opposite side of the laminate; the laminate is positioned in contact with tip 20 such that the electrical pads 380 mate with electrical contacts 70 in the tip. It will be appreciated that in this instance, so-called "backend" processing of the semiconductor chip 30 is required in order to provide the subassembly of the partially encapsulated chip on the laminate. The assembly of semiconductor chip 30 and the laminate component 390 including encapsulated bond wires 365 may be referred to as a semiconductor module 335.

A second arrangement for providing electrical communication between the semiconductor chip and the tip is shown in figure 3(b). In this embodiment, through-silicon-via technology is used such that the semiconductor chip is provided with at least one electrical contact on its second major surface 340, which second major surface is opposed to the first major surface 40. In this embodiment, the electrical contacts 60 may mate directly with the electrical contacts 70 on the tip 20. Such through-silicon-via technology will be well known to the skilled person. In this example arrangement there is a semiconductor module 355 which comprises the semiconductor chip, without any separate laminate or encapsulant.

A third, non-limiting, arrangement for providing electrical communication between the semiconductor chip and the tip is shown in figure 3(c). In this embodiment, in which the chip is embedded in laminate, the electrical contacts 306 from of the chip 30 are connected, by means of interconnects 372, to contact pads 370 on the first surface of laminate 390. The assembly forms a semiconductor module 345. Contact pads 370 are electrically connected to mating contact pads 380 on the opposite surface of the laminate; similar to the embodiment shown in figure 3(a), the mating contact pads 380 are arranged to contact electrical contacts 70 in the tip. Again similar to the arrangement shown in figure 3(a), the semiconductor chip 30 and laminate 390 are at least partially encapsulated by means of encapsulate 375. It will be apparent that encapsulate 375 in neither case encapsulates the electrode or electrodes 50 of the semiconductor chip.

It will thus be appreciated that the semiconductor chip may be comprised in a semiconductor module. In the embodiments shown in figures 3(a) and 3(c), the semiconductor module comprises further components, such as without limitation one or more of the laminate, encapsulant, and wire bonds shown. In other embodiments, the semiconductor module comprises the semiconductor chip alone, such that the semiconductor module is a bare die, or a semiconductor chip including functionalised electrodes. Thus although, in the remaining figures, only the semiconductor chip 30 is shown, it will be appreciated that this may form part of a semiconductor module (345,335), or the whole of a semiconductor module (355).

In order to prevent the analyte coming into contact with electrical contacts 70 or the respective contacts 60 or 380, a sealing element may be provided for providing a seal between the second major surface and the tip. Conveniently such a sealing element may be an O-ring. The sealing element may provide a fluidic seal and may be effective to prevent that the electrical contacts are shorted when the sensor is dipped into the analyte.

Figure 4 shows, at figures 4(a), 4(b) and 4(c) respectively, different stages in a method of sensing for a target molecule in an analyte according to embodiments. At figure 4(a) is shown, schematically, attachment of the tip 20 to the semiconductor chip 30. Semiconductor chip 30 may be positioned in a blister pack 410, or otherwise suitably located. Tip 20 is positioned above chip 30, and then lowered until the chip becomes attached to the tip, by one or more mechanism such as those described above. The apparatus 100, which comprises the assembly of the dipstick having its tip and the chip, is then positioned above a well 420, as is shown in figure 4(b), and lowered into the well. The well 420 is partially filled with analyte 430. If the target molecule (not shown) is present in sufficiently high concentration in the analyte, it is sensed by means of semiconductor chip 30. In order to ensure good fluidic contact between the analyte 430 and the electrode 50 of the semiconductor chip, the apparatus may be agitated with respect to the well. The agitation may, without limitation, comprise a vertical movement, that is a movement up and down, a translation, that is a side-to-side movement, a rotational movement, or a combination of two or more of the above. Once the sensing operation is complete, the apparatus 100 is withdrawn from the well. The semiconductor chip 30 may then be detached from tip 20 and discarded, as shown in figure 4(c). Alternatively, the dipstick may be dipped into a further well containing a washing fluid or a cleaning fluid, prior to possible reuse of the apparatus. This may provide for a conveniently fast throughput, since the time to cleanse the electrodes following a sensing operation may be reduced, relative to that required in a conventional sensing operation in which a fluidic system or a microfluidic system requires to be flushed with washing fluid or cleaning fluid.

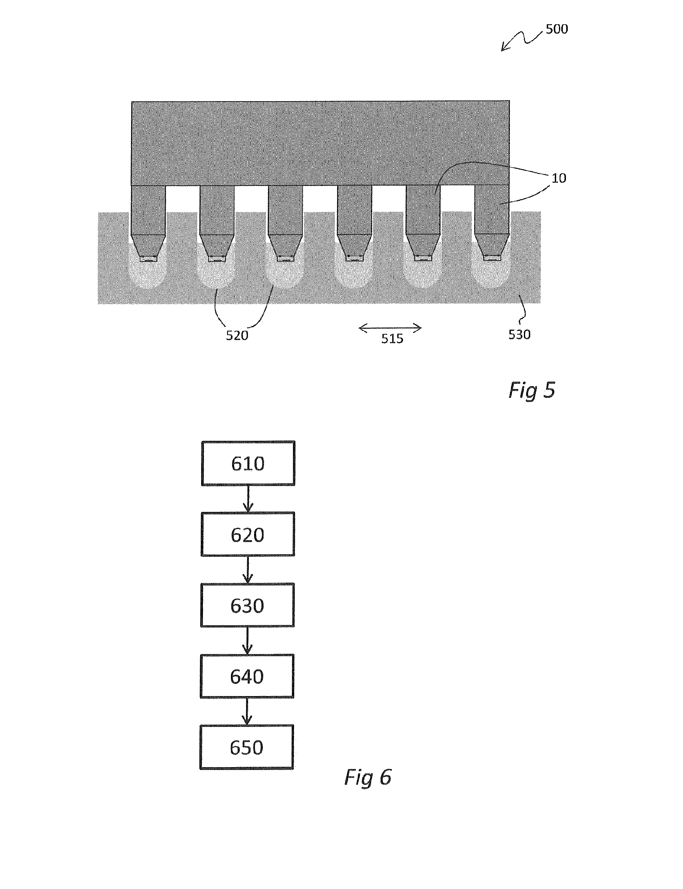

Figure 5 shows an apparatus 500 having a plurality of dipsticks 10. The dipsticks 10 are arranged in an array which may be a linear array as shown, spaced apart by a spacing 515. The spacing 510 may conveniently be regular. The spacing 510 may be chosen such that the dipsticks may be dipped into wells 520 of a microtitre 530 plate, as shown. Microtitre plates are readily available and typically are arranged in standardised arrays, such as a 3:2 rectangular array (examples being 2 rows of 3 wells, 4 rows of 6 wells, 8 rows of 12 wells, and so on). Apparatus according to such an embodiment may thus be compatible with standardised biological assay equipment.

The wells 520 of microtitre plate 530 may have analyte therein. The analyte in individual wells may be the same, or may be different. By using a plurality of different analytes in the different wells 520 of microtitre plate 530, apparatus according to this embodiment may conveniently sense for a particular target molecule in a plurality of analytes, which may be different. In other embodiments the analyte in the different wells 520 of microtitre plate 530 is the same, but the dipsticks 10 of the apparatus are attached to respective chips 30 which are sensitive to different target molecules. Thus in different embodiments, either a plurality of different samples may be analysed for a particular target molecule, or a sample analyte may be analysed for a plurality of different target molecules. A combination of different analytes and different target molecules is also possible.

Figure 6 is a flow diagram of a method according to embodiments of the invention. This method comprises, at 610, removably attaching a semiconductor chip to a tip of a dipstick. The dipstick has a first major surface having at least one electrode thereon, which electrode is sensitive to the target molecule, and a second, opposed, major surface adjoining the tip. At step 620, the tip is dipped into a well having an analyte therein. Then, step 630 comprises electrically sensing for the molecule by means of the semiconductor chip. Thereafter, at step 640, the tip is removed from the well; and at step 650, the semiconductor chip is detached from the tip.

In summary and without limitation, there is disclosed herein a semiconductor chip, apparatus, and associated method wherein the semiconductor chip, having at least one electrode and configured as a sensor such as a biosensor, is removably attachable to a tip of a dipstick. The dipstick tip, with the attached semiconductor chip, is arranged to be dipped into a well containing an analyte. The well may be part of a micro-titre plate. The chip electrically senses the presence of a target molecule, such as a bio-molecule, in the analyte. The sensing may typically be by means of a change in a capacitance associated with the electrode which occurs in the presence of the target molecule. The apparatus may include a plurality of dipsticks and associated semiconductor chips which are sensitive for different target molecules. Alternatively or in addition, a single semiconductor chip may have a plurality, many or even many thousands of electrodes, which may be sensitive to different target molecules.

From reading the present disclosure, other variations and modifications will be apparent to the skilled person. Such variations and modifications may involve equivalent and other features which are already known in the art of sensing for target molecules, and which may be used instead of, or in addition to, features already described herein.

It will be appreciated that although the in description reference has generally been made to biomolecules, as an example subset of large molecules, the invention is not limited to molecules which have biological activity, are organic, or are associated with a living organism.

Although the appended claims are directed to particular combinations of features, it should be understood that the scope of the disclosure of the present invention also includes any novel feature or any novel combination of features disclosed herein either explicitly or implicitly or any generalisation thereof, whether or not it relates to the same invention as presently claimed in any claim and whether or not it mitigates any or all of the same technical problems as does the present invention.

Features which are described in the context of separate embodiments may also be provided in combination in a single embodiment. Conversely, various features which are, for brevity, described in the context of a single embodiment, may also be provided separately or in any suitable sub-combination.

The applicant hereby gives notice that new claims may be formulated to such features and/or combinations of such features during the prosecution of the present application or of any further application derived therefrom.

For the sake of completeness it is also stated that the term "comprising" does not exclude other elements or steps, the term "a" or "an" does not exclude a plurality, and reference signs in the claims shall not be construed as limiting the scope of the claims.

## Claims

1. Apparatus for sensing a target molecule and comprising:
a dipstick having a tip configured to be dipped into a well; and
a semiconductor chip configured as a sensor,
the semiconductor chip having a first major surface having at least one electrode thereon, which electrode is sensitive to the target molecule,
the semiconductor chip being removably attachable to the tip.

2. Apparatus according to claim 1, wherein the semiconductor chip further comprises at least one electrical contact on a second major surface opposed to the first major surface and in electrical communication with the tip.

3. Apparatus according to claim 2, wherein the electrical communication is by means of a resilient element.

4. Apparatus according to any preceding claim, further comprising a sealing element for providing a seal between the second major surface and the tip.

5. Apparatus according to any preceding claim, wherein the semiconductor chip is removably attachable to the tip by at least one of a resilient component and a magnetic field.

6. Apparatus according to any preceding claim, further comprising at least one further dipstick having a respective tip and configured to be dipped into a respective further well, the well and further well being comprised in a microtitre plate.

7. Apparatus according to claim 6,
further comprising a further semiconductor chip being removably attachable to the respective tip and having a major surface having at least one electrode thereon,
which electrode is sensitive to a second target molecule which is different from the target molecule.

8. A semiconductor module comprising a semiconductor chip configured for use in an apparatus as claimed in any of claims 1 to 7.

9. A semiconductor chip as claimed in claim 8 or an apparatus as claimed in any of claims 1 to 7, further comprising at least one further electrode on the first major surface of the semiconductor chip, the at least one further electrode being sensitive to a further target molecule which is different from the target molecule.

10. A method of sensing a target molecule, the method comprising:
removably attaching a semiconductor chip to a tip of a dipstick,
the semiconductor chip having a first major surface having at least one electrode thereon, which electrode is sensitive to the target molecule,
and a second, opposed, major surface adjoining the tip;
dipping the chip into a well having an analyte therein;
electrically sensing for the molecule by means of the semiconductor chip;
removing the chip from the well; and
detaching the semiconductor chip from the tip.

11. The method of claim 10, wherein removably attaching a semiconductor chip to a tip of a dipstick comprises magnetically attaching the chip to the tip.

12. The method of claim 10 or 11, wherein removably attaching a semiconductor chip to a tip of a dipstick further comprises forming a seal, by means of a sealing element, between the second major surface and the tip.

13. The method of any of claims 10 to 12, wherein detaching the semiconductor chip from the tip comprises urging the chip away from the tip by a pin.

14. The method of any of claims 10 to 13, wherein electrically sensing for the target molecule comprises measuring a change in a capacitance associated with the electrode, in the presence of the target molecule.

15. An apparatus according to any of claims 1 to 7, a semiconductor chip according to claim 8 or 9, or the method of any of claims 10-14, wherein the target molecule is a bio-molecule.

## Amended claims

### Amended claims in accordance with Rule 137(2) EPC.

**1.** Apparatus for sensing a target molecule and comprising:
a dipstick (10) having a tip (20) configured to be dipped into a well; and a semiconductor chip (30) configured as a sensor,
the semiconductor chip having a first major surface having at least one electrode (50) thereon, which electrode is sensitive to the target molecule,
the semiconductor chip being removably attachable to the tip.

**2.** Apparatus according to claim 1, wherein the semiconductor chip further comprises at least one electrical contact on a second major surface opposed to the first major surface and in electrical communication with the tip.

**3.** Apparatus according to claim 2, wherein the electrical communication is by means of a resilient element.

**4.** Apparatus according to any preceding claim, further comprising a sealing element for providing a seal between the second major surface and the tip.

**5.** Apparatus according to any preceding claim, wherein the semiconductor chip is removably attachable to the tip by at least one of a resilient component and a magnetic field.

**6.** Apparatus according to any preceding claim, further comprising at least one further dipstick having a respective tip and configured to be dipped into a respective further well, the well and further well being comprised in a microtitre plate.

**7.** Apparatus according to claim 6,
further comprising a further semiconductor chip being removably attachable to the respective tip and having a major surface having at least one electrode thereon,
which electrode is sensitive to a second target molecule which is different from the target molecule.

**8.** A semiconductor module comprising a semiconductor chip configured for use in an apparatus as claimed in any of claims 1 to 7.

**9.** A semiconductor chip as claimed in claim 8 or an apparatus as claimed in any of claims 1 to 7, further comprising at least one further electrode on the first major surface of the semiconductor chip, the at least one further electrode being sensitive to a further target molecule which is different from the target molecule.

**10.** A method of sensing a target molecule, the method comprising:
removably attaching a semiconductor chip to a tip of a dipstick,
the semiconductor chip having a first major surface having at least one electrode thereon, which electrode is sensitive to the target molecule, and a second, opposed, major surface adjoining the tip;
dipping the chip into a well having an analyte therein;
electrically sensing for the molecule by means of the semiconductor chip;
removing the chip from the well; and
detaching the semiconductor chip from the tip.

**11.** The method of claim 10, wherein removably attaching a semiconductor chip to a tip of a dipstick comprises magnetically attaching the chip to the tip.

**12.** The method of claim 10 or 11, wherein removably attaching a semiconductor chip to a tip of a dipstick further comprises forming a seal, by means of a sealing element, between the second major surface and the tip.

**13.** The method of any of claims 10 to 12, wherein detaching the semiconductor chip from the tip comprises urging the chip away from the tip by a pin.

**14.** The method of any of claims 10 to 13, wherein electrically sensing for the target molecule comprises measuring a change in a capacitance associated with the electrode, in the presence of the target molecule.

**15.** An apparatus according to any of claims 1 to 7 or a semiconductor chip according to claim 8 or 9, wherein the target molecule is a biomolecule.
